(19) Europäisches Patentamt
European Patent Office
Office européen des brevets

(11) Veröffentlichungsnummer: **0 189 103**
**B1**

(12) **EUROPÄISCHE PATENTSCHRIFT**

(45) Veröffentlichungstag der Patentschrift: **02.01.91**

(21) Anmeldenummer: **86100451.3**

(22) Anmeldetag: **15.01.86**

(51) Int. Cl.$^5$: **C 07 D 487/04,**
C 07 D 405/12,
C 07 D 403/12,
C 07 D 487/10,
A 61 K 31/415,
A 61 K 31/495, A 61 K 31/50
// (C07D487/04, 235:00,
209:00)

(54) **Neue Pyrrolo-Benzimidazole, Verfahren zu ihrer Herstellung und diese Verbindungen enthaltende Arzneimittel sowie Zwischenprodukte.**

(30) Priorität: **18.01.85 DE 3501497**

(43) Veröffentlichungstag der Anmeldung:
**30.07.86 Patentblatt 86/31**

(45) Bekanntmachung des Hinweises auf die Patenterteilung:
**02.01.91 Patentblatt 91/01**

(84) Benannte Vertragsstaaten:
**AT BE CH DE FR GB IT LI LU NL SE**

(56) Entgegenhaltungen:
**EP-A-0 098 448**
**EP-A-0 161 632**
**DE-A-3 410 168**

**R.C. ELDERFIELD: "Heterocyclic Compounds", Band 3, 1952, Seiten 126-146, John Wiley & Sons, Inc., New York US;**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 116**
**D-6800 Mannheim 31 (DE)**

(72) Erfinder: **Mertens, Alfred, Dr.**
**In der Schanz 31**
**D-6905 Schriesheim (DE)**
Erfinder: **Hölck, Jens-Peter, Dr.rer.nat.**
**Ph. Brunnemer Weg 33**
**D-6800 Mannheim 31 (DE)**
Erfinder: **Berger, Herbert, Dr.Phil.**
**Völklinger Strasse 16**
**D-6800 Mannheim 31 (DE)**
Erfinder: **Müller-Beckmann, Bernd, Dr.**
**Hochgewanne 46**
**D-6718 Grünstadt (DE)**
Erfinder: **Strein, Klaus, Prof.**
**Eichenstrasse 45**
**D-6944 Hemsbach (DE)**
Erfinder: **Roesch, Egon, Dr.med**
**Werderstrasse 44**
**D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Pyrrolo-Benzimidazole der allgemeinen Formel I

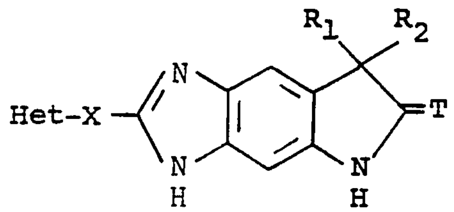

(I)

in welcher

$R_1$ ein Wasserstoffatom, eine $C_1$—$C_6$-Alkyl-, $C_2$—$C_6$-Alkenyl- oder $C_3$—$C_7$-Cycloalkylgruppe bedeutet,

$R_2$ ein Wasserstoffatom, eine $C_1$—$C_6$-Alkyl-, $C_2$—$C_6$-Alkenyl- oder Cyanogruppe, eine durch eine Hydroxy-, $C_1$—$C_6$-Alkyl, $C_1$—$C_6$-Alkoxy-, Amino-, $C_1$—$C_6$-Alkylamino-, Di-$C_1$—$C_6$-alkylamino- oder Hydrazinogruppe substituierte Carbonylgruppe oder mit $R_1$ zusammen eine $C_3$—$C_8$-Cycloalkylengruppe darstellt oder $R_1$ und $R_2$ zusammen eine Alkyliden- oder Cycloalkylidengruppe bilden,

X einen Valenzstrich, eine $C_1$—$C_4$-Alkylengruppe oder die Vinylengruppe bedeutet,

T ein Sauerstoff- oder Schwefelatom ist, und

Het eine Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- oder Tetrazinylrest darstellt und diese Reste durch eine oder mehrere $C_1$—$C_6$-Alkyl-, $C_1$—$C_6$-Alkoxy-, $C_1$—$C_6$-Alkylmercapto, Hydroxy-, Nitro-, Amino-, Halogen- oder Cyangruppen substituiert sein können, deren Tautomere und deren physiologisch verträgliche Salze anorganischer und organischer Säuren und Verfahren zu ihrer Herstellung, sowie diese Verbindungen enthaltende Arzneimittel.

Da die Verbindung der allgemeinen Formel I für den Fall, daß $R_1$ nicht gleich $R_1$ ist, ein asymmetrisches Kohlenstoffatom besitzen, sind auch Gegenstand der Erfindung die optisch aktiven Formen und racemischen Gemische der dieser Verbindungen. Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbessern die Mikrozirkulation.

Aus der früheren europäischen Patentanmeldung EP—A—161,632 sind Verbindungen bekannt, in dene Het eine Pyridylgruppe bedeutet.

In der allgemeinen Formel I stellen die Substituenten $R_1$ und/oder $R_2$ Wasserstoff, eine geradkettige oder verzweigte Alkyl- oder Alkylengruppe mit 1—6 bzw. 2—6 Kohlenstoffatomen, eine Cycloalkylgruppe mit 3—7 Kohlenstoffatomen, eine Cyangruppe oder eine durch eine Hydroxy-, Alkyl-, Alkoxy-, Amino-, Alkylamino-, Dialkylamino- oder Hydrazinogruppe substituierte Carbonylgruppe, wobei der Alkylrest 1—6, vorzugsweise 1—3 Kohlenstoffatome enthält, dar. Insbesondere kommen für $R_1$ und/oder $R_2$ Wasserstoff, die Methyl-, Ethyl-, Isopropyl-, 3-Pentyl-, Cyclopentyl-, Cyclohexyl-, Allyl-, Cyano-, Acetyl-, Propionyl-, Methoxycarbonyl-, Ethoxycarbonyl-, Aminocarbonyl-, Methylaminocarbonyl-, Dimethylaminocarbonyl- und die Hydrazinocarbonylgruppe in Frage.

$R_1$ und $R_2$ können zusammen mit dem C-Atom, an das sie gebunden sind, auch einen Cycloalkylring mit 3—8 Kohlenstoffatomen bilden, vorzugsweise handelt es sich dabei um die Spirocyclopropyl-, Spirocyclobutyl-, Spirocyclopentyl- und Spirocyclohexylgruppe.

$R_1$ und $R_2$ können zusammen auch eine Alkyliden- oder Cycloalkylidengruppe bilden, vorzugsweise handelt es sich dabei um die Isopropylidengruppe.

X bedeutet ein Valenzstrich, eine $C_1$—$C_4$-Alkenylgruppe oder die Vinylengruppe, vorzugsweise jedoch ein Valenzstrich, die Methylen- oder Vinylengruppe.

T ist ein Sauerstoff- oder Schwefelatom, vorzugsweise ein Sauerstoffatom.

Alkyl-, Alkoxy- und Alkylmercapto-Substituenten des Restes Het können 1—6, vorzugsweise 1—4 Kohlenstoffatome enthalten. Bevorzugt ist der Methyl-, Ethyl-, Methoxy-, Ethoxy-, Methylenmercapto- und Ethylmercaptorest. Unter Halogen ist Fluor, Chlor und Brom, vorzugsweise Chlor zu verstehen.

Bevorzugte Substituenten des Restes Het sind Hydroxy, Methyl, Methoxy, Methylmercapto, Amino, Nitro, Halogen oder Cyano, insbesondere Hydroxy, Methyl, Methylmercapto und Cyano.

Die Verbindungen der allgemeinen Formel I können nach den folgenden Schemata 1 und 2 hergestellt werden.

Wie aus dem Schema 1 ersichtlich kann man Verbindungen der allgemeinen Formel II und II durch Nitrierung oder durch Acylierung der Amino-nitro-indolinone IV und V mit einer Verbindung der Formel IX in die Verbindungen der allgemeinen Formel VI und VII überführen, wobei $R_1$, $R_2$, Het und X die angegebene Bedeutung haben. Aus den Verbindungen der Formel VI und VII erhält man durch Reduktion der Nitrogruppe oder aus Verbindungen der Formel VIII durch Umsetzung mit Verbindungen der Formel IX

die Verbindungen der allgemeinen Formel I oder dessen tautomere Form, in der $R_1$, $R_2$, Het und X die angegebene Bedeutung haben.

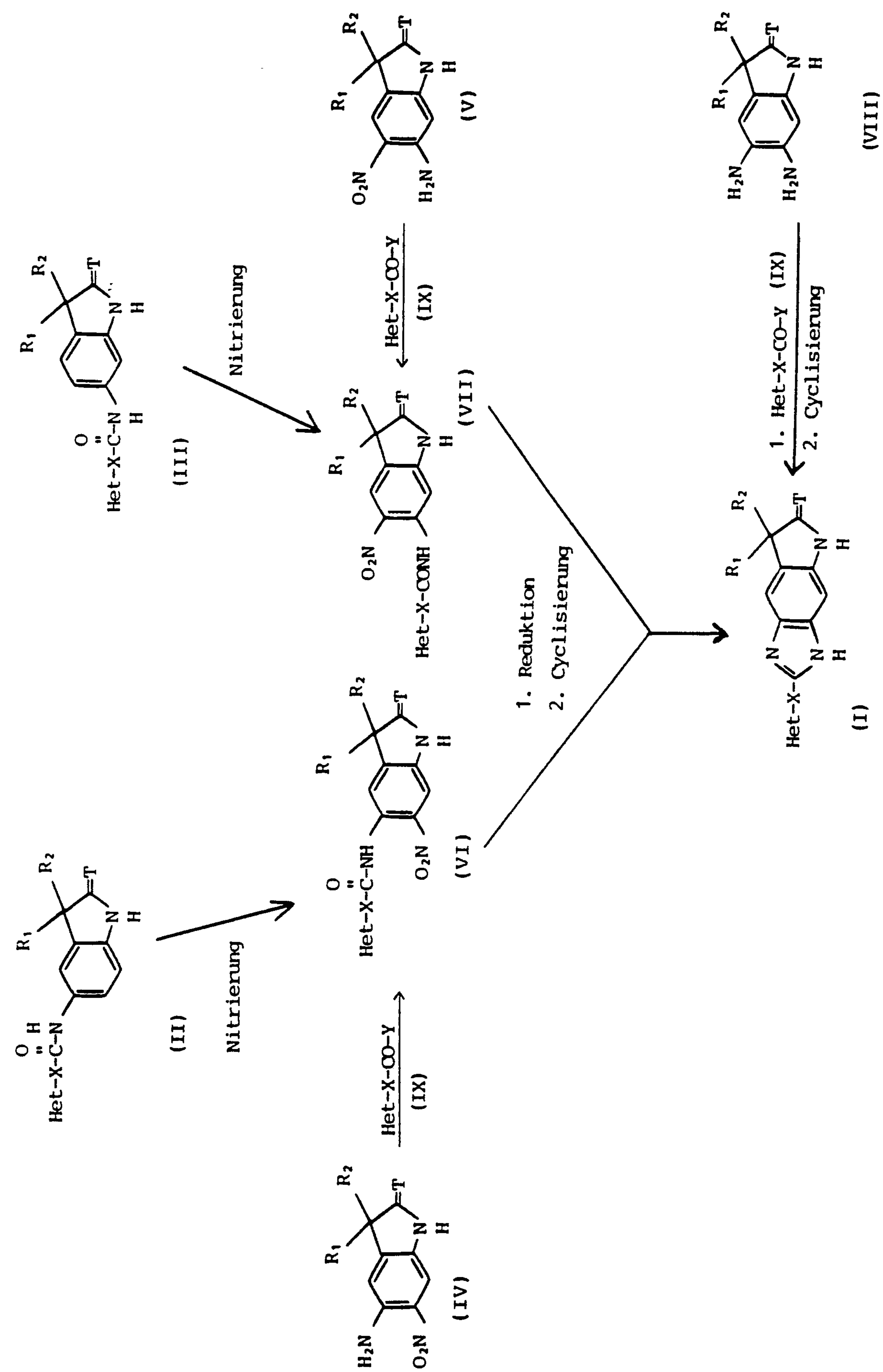


(III) (II) (V) (VII) (VI) (IV) (VIII) (I)

In der allgemeinen Formel IX besitzen Het und X die angegebene Bedeutung und Y ist entweder Wasserstoff oder ein leicht abspaltbarer Rest. Insbesondere verseht man unter den Verbindungen der allgemeinen Formel IX Aldehyde, sowie Saeurehalogenide wie Saeurechloride, Carbonsaeureester wie Methyl- und Ethylester und andere aktivierte Carbonsaeurederivate, wie z.B. Anhydride.

Ist die Verbindung der Formel IX ein Aldehyd findet die Umsetzung zur Schiff'schen Base mit Verbindungen der allgemeinen Formel VIII vorzugsweise in alkoholischem Medium statt, die anschließende Cyclisierung und Oxidation zu den Verbindungen der allgemeinen Formel I erfolgt durch Erwaermen des Reaktionsansatzes zum Rueckfluß in Gegenwart von Luftsauerstoff und katalytischen Mengen Saeure, wie z.B. Toluolsulfonsaeure.

Ist die Verbindung der allgemeinen Formel IX ein Carbonsaeurederivat, findet die Umsetzung mit Verbindungen der allgemeinen Formel VI, VII und VIII zum Amid in inerten Loesungsmitteln, vorzugsweise in Methylenchlorid oder Pyridin statt und die anschließende Cyclisierung zu Verbindungen der allgemeinen Formle I wird nach vorheriger Hydrierung der Nitrogruppe in Verbindungen der Formel VI und VII in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid bei Temperaturen zwischen 0° und 250°C, vorzugsweise jedoch bei der Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Kaliummethylat oder Kalium tert.-butylat durchgefuehrt. Die Cyclisierung kann jedoch auch ohne Loesungsmittel und/oder Kondensationsmittel durchgefuehrt werden.

Die oben erwaehnte Hydrierung der Nitrogruppe wird vorzugsweise in einem Loesungsmittel wie Wasser, Ethanol, Eisessig, Essigsaeureethylester oder Dimethylformamid zweckmaeßigerweise mit Wasserstoff in Gegenwart eines Hydrierungskatalysators wie Raney-Nickel, Platin oder Palladium/Kohle, mit Metallen wie Eisen(II)sulfat, Zinn(II)chlorid, Natriumsulfid, Natriumhydrogensulfid oder Natriumdithionit, oder mit Hydrazin in Gegenwart von Raney-Nickel bei Temperaturen zwischen 0 und 250°C, vorzugsweise jedoch bei Raumtemperatur, durchgefuehrt.

Die Verbindungen IV, V und VIII sind literaturbekannt (vgl. Anmeldung P 34 17 643.8). Die Verbindungen VI und VII sind neu und ebenfalls Gegenstand der Erfindung.

Wie aus dem Schema 2 ersichtlich, lassen sich die Verbindungen der allgemeinen Formel I oder dessen tautomere Formen auch nach verschiedenen literaturbekannten Verfahren aus Verbindungen der Formel X, in der Het und X die angegebene Bedeutung haben, herstellen (vgl. hierzu Elderfield, R. C. (Ed.); Julian, P. L.; Meyer, E. W.; Printy, H. C.; Heterocyclic Compounds Vol. 3, 126—142, John Wiley & Sons 1952, New York).

*Hinsberg Synthese:* Umsetzung aromatischer Amine mit den Bisulfit-Additions-Verbindungen von Ketonen.

*Brunner Synthese:* Cyclisierung aromatischer Amine über das Hydrazit zu Oxindolen.

*Stollé Synthese:* Cyclisierung aromatischer Amine über ein Amid zum Oxindol.

Schema 2

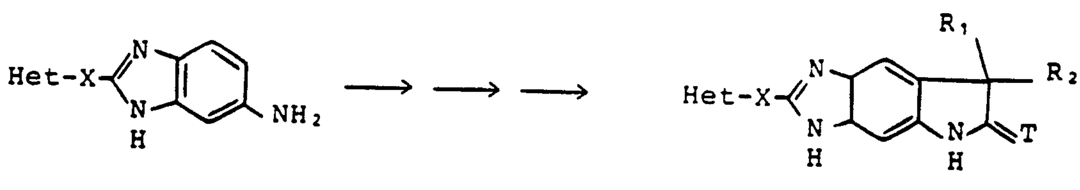

(X) (I)

Die Verbindungen der Formel X sind zum Teil neu und koennen nach literaturbekannten Verfahren hergestellt werden.

Verbindungen der allgemeinen Formel I koennen auch nachtraeglich in eine andere Verbindung der Formel I umgewandelt werden. Dies trifft zum Beispiel zu

a) fuer die Oxidation eines Feunf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoroessigsaeure oder in Ameisensaeure bie 20—100°C oder in Aceton bei 0—60°C, mit einer Persaeure wie Perameisensaeure oder m-Chlorperbenzoesaeure in Eisessig, Trifluoroessigsaeure, Methylenchlorid oder Chloroform bei Temperturen zwischen 0 und 60°C.

b) fuer die Hydrierung einer Vinylenverbindung (X = —CH=CH—) in eine entsprechende Ethylenverbindung (X = $—CH_2—CH_2—$). Die Hydrierung wird vorzugsweise in einem Loesungsmittel wie Wasser, Wasser/Ethanol, Methanol, Eisessig, Essigsaeureethylester oder Dimethylformamid zweckmaßigerweise mit Wasserstoff in Gegenwart eines Hydrierungskatalysators wie Raney-Nickel, Platin oder Palladium/Kohle durchgefuehrt.

c) fuer die Umsetzung einer Verbindung der Formel I, in der $R_1$ und $R_2$ gleich Wasserstoff sind, mit einer Verbindung der Formel XI

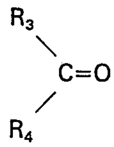

(XI)

in der $R_3$ und $R_4$ Alkylgruppen sind oder $R_3$ zusammen mit $R_4$ eine $C_3$—$C_7$-Cycloalkylengruppe bildet, zu einer neuen Verbindung der allgemeinen Formel I, in der $R_1$ zusammen mit $R_2$ eine Alkyliden- oder Cycloalkylidengruppe darstellt, sowie gegebenenfalls deren Hydrierung zu den entsprechenden Verbindungen der allgemeinen Formel I, in der $R_1$ oder $R_2$ gleich Wasserstoff ist.

Die Umsetzung mit Verbindungen der Formel XI wird vorzugsweise in polaren Loesungsmitteln wie z.B. Ethanol oder Dimethylformamid in Gegenwart einer Base wie z.B. Ammoniak oder Triethylamin oder auch in Gegenwart einer Saeure wie Salzsaeure, Schwefelsaeure, Phosphorsaeure, Polyphosphorsaeure oder p-Toluolsulfonsaeure bei 20—250°C vorzugsweise jedoch bei der Siedetemperatur des Loesungsmittels durchgefuehrt.

Die gegebenenfalls auszufuehrende Hydrierung wird wie bei den unter b) beschriebenen Bedingungen durchgefuehrt.

d) fuer die Umsetzung von Verbindungen der allgemeinen Formel I, in der $R_1$ oder $R_2$ eine Carboxylgruppe darstellt, oder ein reaktionsfaehiges Derivat hiervon, wie z.B. Ester oder Saeurechlorid, mit Hydrazin oder einem Amin der allgemeinen Formel XII,

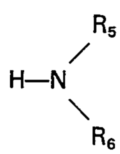

(XII)

in der $R_5$ und $R_6$, die gleich oder verschieden sein koennen, Wasserstoffatome oder Alkylgruppen mit 1—5 Kohlenstoffatomen darstellen, oder mit einem reaktionsfaehigen Derivat hiervon, falls $R_1$ oder $R_2$ die Carboxylgruppe ist, zu neuen Verbindungen der allgemeinen Formel I, in der $R_1$ oder $R_2$ eine durch eine Amino-, Alkylamino-, Dialkylamino- oder Hydrazinogruppe substituierte Carbonylgruppe ist.

Die Umsetzung wird zweckmaeßigerweise in einem Loesungsmittel oder Loesungsmittelgemisch wie Methylenchlorid, Ethanol, Chloroform, Tetrachlorokohlenstoff, Ether, Tetrahydrofuran, Dioxan, Benzol, Acetonitril oder Dimethylformamid gegebenenfalls in Gegenwart eines die Saeure aktivierenden Mittels oder eines wasserentziehenden Mittels, z.B. in Gegenwart von Chlorameisensaeureethylester, Thionylchlorid, Phosphortrichlorid, Phosphorpentoxid, N',N-Dicyclohexylcarbodiimid, N',N-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Hydrazino- oder Aminogruppe aktivierenden Mittels, z.B. Phosphortrichlorid, und gegebenenfalla in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen −25 und 250°C, vorzugsweise jedoch bei Temperaturen zwischen −10°C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsaeurechlorid, und Hydrazin oder einem entspechenden Amin, wobei diese gleichzeitig als Loesungsmittel dienen koennen, und bei Temperaturen zwischen 0 und 50°C durchgefuehrt.

e) fuer die Umsetzung einer Verbindung der allgemeinen Formel I, in der $R_1$ oder $R_2$ eine Aminocarbonylgruppe darstellt, zu einer anderen Verbindung der allgemeinen Formel I, in der $R_1$ oder $R_2$ eine Cyangruppe ist.

Die Umsetzung wird zweckmaeßigerweise in einem inerten Loesungsmittel, wie z.B. in Methylenchlorid, Chloroform, Dioxan, Pyridin, Xylol oder Chlorbenzol in Gegenwart eines wasserentziehenden Mittels, wie z.B. Thionylchlorid, Phosphortrichlorid, Phosphorpentoxid, Phosphorpentachlorid, Aluminiumchlorid, Benzolsulfonsaeurechlorid, Toluolsulfonsaeurechlorid, Triphenylphosphin, Bortrifluorid oder Polyphosphorsaeureethylester bei Temperaturen zwischen 50 und 250°C vorzugsweise jedoch bei der Siedetemperatur des Loesungsmittels durchgefuehrt.

f) fuer die Umsetzung einer Verbindung der allgemeinen Formel I, in der $R_1$ oder $R_2$ die Cyangruppe darstellt, zu einer anderen Verbindung der allgemeinen Formel I, in der $R_1$ oder $R_2$ eine Carboxyl-, Aminocarbonyl- oder Alkoxycarbonylgruppe mit insgesamt 1—6 Kohlenstoffatomen bilden.

Diese Alkoholyse und/oder Hydrolyse wird zweckmaeßigerweise in Gegenwart einer Saeure wie Salzsaeure, Schwefelsaeure, Phosphorsaeure oder Trichloressigsaeure oder in Gegenwart einer Base wie

Natriumhydroxid oder Kaliumhydroxid in einem geeigneten Loesungsmittel wie Wasser, Wasser/Methanol, Ethanol, Wasser/Isopropanol oder Wasser/Dioxan zwischen −10 und 120°C, z.B. bei Temperaturen zwischen Raumtemperatur und der Siedetemperatur des Reaktionsgemisches, durchgefuehrt.

g) fuer die Umsetzung einer Verbindung der allgemeinen Formel I, in der T = Sauerstoff ist, zu einer anderen Verbindung der allgemeinen Formel I, in der T = Schwefel ist.

Die Umsetzung wird nach literaturbekannten Verfahren mit einem das Schwefelatom uebertragenden Reagenz, wie z.B. Phosphorpentasulfid durchgefuehrt, wobei man zweckmaeßigerweise 1—5 Mol $P_2S_5$ vorzugsweise jedoch 1 Mol in einem geeigneten Loesungsmittel umsetzt. Als Loesungsmittel kommen Tetrahydrofuran, Dioxan, Benzol, Toluol oder Pyridin bei Temperaturen zwischen 25 und 125°C in Betracht. Bevorzugt ist jedoch Pyridin bei einer Reaktionsdauer von 1—10 Stunden, vorzugsweise jedoch 5 Stunden in Abhaengigkeit vom Reaktionspartner.

Ferner koennen die erhaltenen Verbindungen der allegemeinen Formel I anschließend gewuenschtenfalls in ihre physiologisch vertraeglichen Saeureadditionssalze mit anorganischen und organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nichttoxischen Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueberlicherweise in Mengen von 10—500 mg pro Tag bezogen auf 75 kg Koerpergewicht appliziert. Bevorzugt ist es, 2—3 mal pro Tag 1—2 Tabletten mit einem Wirkstoffgehalt von 5—200 mg zu verabreichen. Die Tabletten koennen auch retardiert sein, wodurch nur noch 1 mal pro Tag 1—2 Tabletten mit 10—500 mg Wirkstoff gegeben werden muß. Der Wirkstoff kann auch durch Injektion 1—8 mal pro Tag bzw. durch Dauerinfusion gegeben werden, wobei Mengen von 5—200 mg/Tag normalerweise ausreichen.

Wie bereits erwaehnt sind die Verbindungen VI und VII in Schema 1 ebenfalls neu und Gegenstand der Erfindung.

Erfindungsmaeßige Verbindungen der Formeln VI und VII sind außer den in den Beispielen genannten Verbindungen die folgenden Verbindungen:

6-Nitro-5-(2-furanyl-carbonylamino)-indolin-2-on
6-Nitro-5-(3-pyridazinyl-carbonylamino)-3,3-dimethyl-indolin-2-on
6-Nitro-5-[(6-methyl-pyrimidin-4-yl)carbonylamino]-3,3-dimethyl-indolin-2-on
6-Nitro-5-[(1,2,5-thiadiazol-3-yl)carbonylamino]-3,3-dimethyl-indolin-2-on
6-Nitro-5-[(1,3,4-thiadiazol-2-yl)carbonylamino]-3,3-dimethyl-indolin-2-on
6-Nitro-5-(2-furanyl-carbonylamino)-3,3-diethyl-indolin-2-on
6-Nitro-5-(4-pyridazinyl-carbonylamino]-3-isopropyliden-indolin-2-on
5-Nitro-6-[(2-hydroxy-pyrimidin-5-yl)carbonylamino]-3-methyl-indolin-2-on
5-Nitro-6-[(3,6-dimethyl-pyrimidin-4-yl)carbonylamino]-3-methyl-indolin-2-on
5-Nitro-6-[(1-methyl-2-oxo-pyrimidin-5-yl)carbonylamino]-3-ethyl-indolin-2-on
5-Nitro-6-[(1,2,5-oxadiazol-3-yl)carbonylamino]-3-cyclopentyl-indolin-2-on
5-Nitro-6-(3-pyridazinyl-carbonylamino)-3-methyl-3-ethoxy-carbonyl-indolin-2-on
5-Nitro-6-(4-pyrimidinyl-carbonylamino)-3-methyl-3-ethoxycarbonyl-indolin-2-on
5-Nitro-6-(2-pyrazinyl-carbonylamino)-3-methyl-3-ethoxycarbonyl-indolin-2-on
5-Nitro-6-[(2-methyl-pyrimidin-5-yl)-carbonylamino)-3-methyl-3-ethoxycarbonyl-indolin-2-on
5-Nitro-6-[(1-methyl-2-oxo-pyrimidin-5-yl)carbonylamino]-3-methyl-3-ethoxycarbonyl-indolin-2-on
5-Nitro-6-[(6-methyl-pyrimidin-4-yl)carbonylamino]-3-methyl-3-ethoxycarbonyl-indolin-2-on
5-Nitro-6-[(6-hydroxy-pyridazin-3-yl)-carbonylamino]-3-ethoxycarbonyl-indolin-2-on
5-Nitro-6-[(3,6-dimethyl-pyridazin-4-yl)carbonylamino]-3-ethoxycarbonyl-indolin-2-on
5-Nitro-6-[(1,3,5-thiadiazol-2-yl)carbonylamino]-3-ethoxycarbonyl-indolin-2-on
5-Nitro-6-[(2-methyl-pyrimidin-5-yl)carbonylamino]-3-methyl-3-acetyl-indolin-2-on

Neue Verbindungen der allgemeinen Formel I sind außer den in den Beispielen genannten Verbindungen die folgenden und deren Tautomere:

7,7-Dimethyl-2-(3-pyridazinyl)-6,7-dihydro-3H,5H-pyrrolo[2,3-f]benzimidazol-6-on
7,7-Dimethyl-2-(6-methyl-pyrmidin-4-yl)-6,7-dihydro-3H,5H-pyrrolo[2,3-f]benzimidazol-6-on
7,7-Dimethyl-2-(3-pyrazolyl)-6,7-dihydro-3H,5H-pyrrolo[2,3-f]benzimidazol-6-on
7,7-Dimethyl-2-(4-pyrazolyl)-6,7-dihydro-3H,5H-pyrrolo[2,3-f]benzimidazol-6-on
7,7-Dimethyl-2-(2-thienyl-methyl)-6,7-dihydro-3H,5H-pyrrolo[2,3-f]benzimidazol-6-on
7,7-Dimethyl-2-(2,6-dihydroxy-pyrimidin-4-yl)-6,7-dihydro-3H,5H-pyrrolo[2,3-f]benzimidazol-6-on
7,7-Dimethyl-2-(4-oxazolyl)-6,7-dihydro-3H,5H-pyrrolo[2,3-f]benzimidazol-6-on
7,7-Dimethyl-2-(1,3,4-thiadiazol-2-yl)-6,7-dihydro-3H,5H-pyrrolo[2,3-f]benzimidazol-6-on
7,7-Dimethyl-2-(1,2,3-thiadiazol-4-yl)-6,7-dihydro-3H,5H-pyrrolo[2,3-f]benzimidazol-6-on, Mp. >300°C aus Isopropanol
7,7-Dimethyl-2-(1,2,3-thiadiazol-5-yl)-6,7-dihydro-3H,5H-pyrrolo[2,3-f]benzimidazol-6-on
7,7-Dimethyl-2-(2-methylmercapto-1,3,4-oxadiazol-5-yl)-6,7-dihydro-3H,5H-pyrrolo[2,3-f]benzimidazol-6-on
7,7-Dimethyl-2-(4-carboxy-1,2,3-1H-triazol-5-yl)-6,7-dihydro-3H,5H-pyrrolo[2,3-f]benzimidazol-6-on
7,7-Dimethyl-2-(4-methoxycarbonyl-1,2,3-1H-triazol-5-yl)-6,7-dihydro-3H,5H-pyrrolo[2,3-f]benzimidazol-6-on
7,7-Dimethyl-2-(4-aminocarbonyl-1,2,3-1H-triazol-5-yl)-6,7-dihydro-3H,5H-pyrrolo[2,3-f]benzimidazol-6-on
7,7-Dimethyl-2-(4-cyan-1,2,3-1H-triazol-5-yl)-6,7-dihydro-3H,5H-pyrrolo[2,3-f]benzimidazol-6-on
7,7-Dimethyl-2-(5,6-dimethyl-1,2,4-triazin-3-yl)-6,7-dihydro-3H,5H-pyrrolo[2,3-f]benzimidazol-6-on
7,7-Diethyl-2-(2-furanyl)-6,7-dihydro-3H,5H-pyrrolo[2,3-f]benzimidazol-6-on
2'-(2-Furanyl)spiro[cyclopentan-1,7'—6',7'-dihydro-3'H,5'H-pyrrolo[2',3'-f]benzimidazol]-6'-on, Mp. >300°C aus Methylethylketon
2'-(4-pyridazinyl)-spiro[cyclopentan-1,7'—6',7'-dihydro-3'H,5'H-pyrrolo[2',3'-f]benzimidazol]-6'-on
7-Methyl-2-(2-pyrrolyl)-6,7-dihydro-3H,5H-pyrrolo[2,3-f]benzimidazol-6-on
7-Methyl-2-(2-hydroxy-pyrimidin-5-yl)-6,7-dihydro-3H,5H-pyrrolo[2,3-f]benzimidazol-6-on
7-Methyl-2-(3,6-dimethyl-pyridazin-4-yl)-6,7-dihydro-3H,5H-pyrrolo[2,3-f]benzimidazol-6-on
7-Methyl-2-(2-oxo-pyran-5-yl)-6,7-dihydro-3H,5H-pyrrolo[2,3-f]benzimidazol-6-on
7-Methyl-2-(2,6-dihydroxy-pyrimidin-4-yl)-6,7-dihydro-3H,5H-pyrrolo[2,3-f]benzimidazol-6-on
7-Methyl-2-(2-oxazolyl)-6,7-dihydro-3H,5H-pyrrolo[2,3-f]benzimidazol-6-on
7-Methyl-2-(1,3,5-triazin-2-yl)-6,7-dihydro-3H,5H-pyrrolo[2,3-f]benzimidazol-6-on
7-Ethyl-2-(4-imidazolyl)-6,7-dihydro-3H,5H-pyrrolo[2,3-f]benzimidazol-6-on
7-Ethyl-2-(1-methyl-2-oxo-pyrimidin-5-yl)-6,7-dihydro-3H,5H-pyrrolo[2,3-f]benzimidazol-6-on
7-Ethyl-2-(2-imidazolyl)-6,7-dihydro-3H,5H-pyrrolo[2,3-f]benzimidazol-6-on
7-Ethyl-2-(3-methyl-1,2,4-triazin-5-yl)-6,7-dihydro-3H,5H-pyrrolo[2,3-f]benzimidazol-6-on
7-Ethyl-2-(2-thiazolyl)-6,7-dihydro-3H,5H-pyrrolo[2,3-f]benzimidazol-6-on
7-Ethyl-2-(1,2,4-triazin-5-yl)-6,7-dihydro-3H,5H-pyrrolo[2,3-f]benzimidazol-6-on
7-Ethyl-2-(4-pyridazinyl)-6,7-dihydro-3H,5H-pyrrolo[2,3-f]benzimidazol-6-on
7-Isopropyl-2-(4-thiazolyl)-6,7-dihydro-3H,5H-pyrrolo[2,3-f]benzimidazol-6-on
7-Isopropyl-2-(1,2,4,5-tetrazin-3-yl)-6,7-dihydro-3H,5H-pyrrolo[2,3-f]benzimidazol-6-on
7-Cyclopentyl-2-(5-thiazolyl)-6,7-dihydro-3H,5H-pyrrolo[2,3-f]benzimidazol-6-on
7-Cyclopentyl-2-(1,2,5-oxadiazol-3-yl)-6,7-dihydro-3H,5H-pyrrolo[2,3-f]benzimidazol-6-on
7-Isopropyliden-2-(4-pyridazinyl)-6,7-dihydro-3H,5H-pyrrolo[2,3-f]benzimidazol-6-on
7-Isopropyliden-2-(1H-tetrazol-5-yl)-6,7-dihydro-3H,5H-pyrrolo[2,3-f]benzimidazol-6-on
2-(2-Furanyl)-6,7-dihydro-3H,5H-pyrrolo[2,3-f]benzimidazolo-6-on
7-Methyl-7-ethoxycarbonyl-2-(3-pyridazinyl)-6,7-dihydro-3H,5H-pyrrolo[2,3-f]benzimidazol-6-on
7-Methyl-7-ethoxycarbonyl-2-(4-pyrimidinyl)-6,7-dihydro-3H,5H-pyrrolo[2,3-f]benzimidazol-6-on
7-Methyl-7-ethoxycarbonyl-2-(2-pyrazinyl)-6,7-dihydro-3H,5H-pyrrolo[2,3-f]benzimidazol-6-on
7-Methyl-7-ethoxycarbonyl-2-(2-hydroxy-pyrimidin-5-yl)-6,7-dihydro-3H,5H-pyrrolo[2,3-f]benzimidazol-6-on
7-Methyl-7-ethoxycarbonyl-2-(1-methyl-2-oxo-pyrimidin-5-yl)-6,7-dihydro-3H,5H-pyrrolo[2,3-f]benzimidazol-6-on
7-Methyl-7-ethoxycarbonyl-2-(6-methyl-pyrimidin-4-yl)-6,7-dihydro-3H,5H-pyrrolo[2,3-f]benzimidazol-6-on
7-Methyl-7-ethoxycarbonyl-2-(5-oxazolyl)-6,7-dihydro-3H,5H-pyrrolo[2,3-f]benzimidazol-6-on
7-Methyl-7-ethoxycarbonyl-2-(2-thiazolyl)-6,7-dihydro-3H,5H-pyrrolo[2,3-f]benzimidazol-6-on
7-Methyl-7-ethoxycarbonyl-2-(4-pyrazolyl)-6,7-dihydro-3H,5H-pyrrolo[2,3-f]benzimidazol-6-on
7-Methyl-7-ethoxycarbonyl-2-(1,2,4-triazin-5-yl)-6,7-dihydro-3H,5H-pyrrolo[2,3-f]benzimidazol-6-on
7-Methyl-7-ethoxycarbonyl-2-(5-methyl-pyrazol-3-yl)-6,7-dihydro-3H,5H-pyrrolo[2,3-f]benzimidazol-6-on
7-Methyl-7-aminocarbonyl-2-(4-pyrimidinyl)-6,7-dihydro-3H,5H-pyrrolo[2,3-f]benzimidazol-6-on
7-Methyl-7-aminocarbonyl-2-(5-oxazolyl)-6,7-dihydro-3H,5H-pyrrolo[2,3-f]benzimidazol-6-on
7-Methyl-7-methylaminocarbonyl-2-(1-methyl-2-oxo-pyrimidin-5-yl)-6,7-dihydro-3H,5H-pyrrolo[2,3-f]benzimidazol-6-on

7-Methyl-7-methylaminocarbonyl-2-(1,3,4-triazin-5-yl)-6,7-dihydro-3H,5H-pyrrolo[2,3-f]benzimidazol-6-on

7-Methyl-7-dimethylaminocarbonyl-2-(6-methyl-pyrimidin-4-yl)-6,7-dihydro-3H,5H-pyrrolo[2,3-f]benzimidazol-6-on

7-Methyl-7-methylaminocarbonyl-2-(1,3,4-triazin-5-yl)-6,7-dihydro-3H,5H-pyrrolo[2,3-f]benzimidazol-6-on

7-Methyl-7-dimethylaminocarbonyl-2-(6-methyl-pyrimidin-4-yl)-6,7-dihydro-3H,5H-pyrrolo[2,3-f]benzimidazol-6-on

7-Methyl-7-dimethylaminocarbonyl-2-(5-methyl-pyrazol-3-yl)-6,7-dihydro-3H,5H-pyrrolo-[2,3-f]benzimidazol-6-on

7-Methyl-7-cyan-2-(2-pyrazinyl)-6,7-dihydro-3H,5H-pyrrolo[2,3-f]benzimidazol-6-on

7-Methyl-7-cyan-2-(2-thiazolyl)-6,7-dihydro-3H,5H-pyrrolo[2,3-f]benzimidazol-6-on

7-Methyl-7-hydrazinocarbonyl-2-(2-methyl-pyrimidin-5-yl)-6,7-dihydro-3H,5H-pyrrolo[2,3-f]benzimidazol-6-on

7-Methyl-7-hydrazinocarbonyl-2-(4-pyrazolyl)-6,7-dihydro-3H,5H-pyrrolo[2,3-f]benzimidazol-6-on

7-Ethoxycarbonyl-2-(1,2,4-triazol-3-yl)-6,7-dihydro-3H,5H-pyrrolo[2,3-f]benzimidazol-6-on

7-Ethoxycarbonyl-2-(3-pyrazolyl)-6,7-dihydro-3H,5H-pyrrolo[2,3-f]benzimidazol-6-on

7-Ethoxycarbonyl-2-(1,2,5-thiadiazol-3-yl)-6,7-dihydro-3H,5H-pyrrolo[2,3-f]benzimidazol-6-on

7-Ethoxycarbonyl-2-(2-oxo-pyrain-5-yl)-6,7-dihydro-3H,5H-pyrrolo[2,3-f]benzimidazol-6-on

7-Ethoxycarbonyl-2-(1,3,4-thiadiazol-2-yl)-6,7-dihydro-3H,5H-pyrrolo[2,3-f]benzimidazol-6-on

7-Aminocarbonyl-2-(3,6-dimethyl-pyridazin-4-yl)-6,7-dihydro-3H,5H-pyrrolo[2,3-f]benzimidazol-6-on

7-Methylaminocarbonyl-2-(1,2,5-thiadiazol-3-yl)-6,7-dihydro-3H,5H-pyrrolo[2,3-f]benzimidazol-6-on

7-Dimethylaminocarbonyl-2-(2-oxo-pyran-5-yl)-6,7-dihydro-3H,5H-pyrrolo[2,3-f]benzimidazol-6-on

7-Cyan-2-(1,2,4-triazol-3-yl)-6,7-dihydro-3H,5H-pyrrolo[2,3-f]benzimidazol-6-on

7-Hydrazinocarbonyl-2-(3-pyrazolyl)-6,7-dihydro-3H,5H-pyrrolo[2,3-f]benzimidazol-6-on

7-Acetyl-2-(2-imidazolyl)-6,7-dihydro-3H,5H-pyrrolo[2,3-f]benzimidazol-6-on

### Beispiel 1

7,7-Dimethyl-2-(2-furanyl)-6,7-dihydro-3H,5H-pyrrolo[2,3-f]benzimidazol-6-on

a) In eine Loesung von 5.5 g (25 mmol) 6-Amino-5-nitro-3,3-dimethyl-indolin-2-on in 50 ml Pyridin werden unter Eiskuehlung 6.5 g (50 mmol) Furan-2-carbonsaeurechlorid zugetropft und bei 25°C nachgeruehrt. Der Kristallbrei wird auf ca. 300 ml Wasser gegossen, abgesaugt, mit Wasser gewaschen und getrocknet. Das Rohprodukt wird ohne Reinigung weiterverarbeitet.

Ausbeute: 10.2 g 6-Furanoylamino-5-nitro-3,3-dimethyl-indolin-2-on; Mp. 229—35°C.

b) Zu 10.0 g (31.7 mmol) 6-(2-Furanyl-carbonylamino)-5-nitro-3,3-dimethyl-indolin-2-on in 250 ml Ethanol werden 1.0 g Palladium auf Kohle (10 proz.) zugesetzt. Diese Suspension wird in einer Schuettelente bei Zimmertemperatur und Normaldruck hydriert. Nach beendeter Wasserstoffaufnahme wird die Loesung vom Katalysator abgetrennt und zur Trockene eingedampft. Das Rohprodukt wird ohne Reinigung weiterverarbeitet.

Ausbeute: 9.5 g 6-Furanoylamino-5-amino-3,3-dimethyl-indolin-2-on; Mp. 215—224°C.

c) 5.7 g (20 mmol) 6-Furanoylamino-5-amino-3,3-dimethyl-indolin-2-on werden in 200 ml Ethanol und 40 ml conc. Salzsaeure eine Stunde auf 80°C erwaermt. Die Loesung wird zur Trockene gebracht, mit 2 N Ammoniak alkalisch gestellt und mit Methylenchlorid extrahiert. Die organische Phase wird eingeengt und der Rueckstand ueber Kieselgel (Laufmittel: Methylenchlorid/$NH_3$ gesaettigtes Methanol 20:1) gereinigt.

Ausbeute: 1.8 g (33.3% d.Th.); Mp. 311—316°C aus Essigester.

### Beispiel 2

7,7-Dimethyl-2-(2-thienyl)-6,7-dihydro-3H,5H-pyrrolo[2,3-f]benzimidazol-6-on

Eine Loesung von 3.8 g (20 mmol) 5,6-Diamino-3,3-dimethyl-indolin-2-on, 2.25 g (20 mmol) Thiophen-2-aldehyd und 4 ml Eisessig in 40 ml Ethanol wird eine Stunde zum Rueckfluß erwaermt und eine weitere Stunde unter Luftdurchleiten weitergekocht. Der Ansatz wird zur Trockne destilliert und der Rueckstand mit Essigester durchgeruehrt und abgesaugt. Das Krisallisat wurde aus Aceton umkristallisiert.

Ausbeute: 1.7 g (30% d.Th.); Mp. 332—336°C.

### Beispiel 3

7,7-Dimethyl-2-(2-pyrrolyl)-6,7-dihydro-3H,5H-pyrrolo[2,3-f]benzimidazol-6-on

Analog zu Beispiel 2 erhaelt man aus 3.8 g (20 mmol) 5,6-Diamino-3,3-dimethyl-indolin-2-on, 1.9 g (20 mmol) Pyrrol-2-aldehyd und 0.4 g (2 mmol) p-Toluolsulfonsaeure nach Ansaeuren und Eindampfen des Ansatzes das Rohprodukt der Titelverbindung. Zur Reinigung wurde der Rueckstand mit Wasser durchgearbeitet, das Filtrat mit 2 N Ammoniak neutralisiert und abgesaugt. Der Rueckstand wurde aus Isopropanol unter Zusatz von ethanolischer Salzsaeure umkristallisiert.

Ausbeute: 1.2 g (20% d.Th.); Mp. >300°C Hydrochlorid.

Beispiel 4

7,7-Dimethyl-2-(2-pyrazinyl)-6,7-dihydro-3H,5H-pyrrolo[2,3-f]benzimdazolo-6-on

a) 5.1 g (26 mmol) 5,6-Diamino-3,3-dimethyl-indolin-2-on werden in 100 ml Methylenchlorid geloest, mit 5.5 g (39 mmol) Pyrazin-2-carbonsaeurechlorid und 3.9 g (39 mmol) Triethylamin versetzt. Nach einer Stunde bei 25°C wird das Loesungsmittel abdestilliert, der Rueckstand mit Wasser durchgearbeitet und abgesaugt. Der Rueckstand aus Mono- und Diamid wurde aus Essigester/Methanol umkristallisiert.

Ausbeute: 5.6 g; Mp. 269—272°C.

b) 5.6 g des Mono- und Diamids wurden mit 85 ml Ethanol und 85 ml conc. Salzsaeure 18 Stunden bei 90°C geruehrt. Anschließend wurde der Ansatz eingeengt, mit 2 N Ammoniak neutralisiert und abgesaugt. Der Rueckstand wurde ueber Kieselgel (Laufmittel: Methylenchlorid/Methanol 19:1) gereinigt und aus Essigester/Methanol umkristallisiert.

Ausbeute: 0.85 g (16% d.Th.); Mp. >300°C.

Beispiel 5

7,7-Dimethyl-2-(4-thiazolyl)-6,7-dihydro-3H,5H-pyrollo[2,3-f]benzimidazol-6-on

Analog zu Beispiel 4 wurde ausgehend von 3.8 g (20 mmol) 5,6-Diamino-3,3-dimethyl-indolin-2-on und 4.4 g (30 mmol) Thiazol-4-carbonsaeurechlorid die Titelverbindung erhalten.

Ausbeute: 1.9 g (33% d.Th); Mp. 280°C aus Methanol.

Beispiel 6

7,7-Dimethyl-2-(4-pyridazinyl)-6,7-dihydro-3H,5H-pyrrolo-[2,3-f]benzimidazol-6-on

a) 5.4 (40 mmol) 1-Hydroxy-1H-benzotriazol wird mit 5 g $CaSO_4$ und 4.3 g (35 mmol) Pyridazin-4-carbonsaeure in absolutem Dimethylformamid vorgelegt. Bei 0°C wird 8.3 g (40 mmol) N,N-Dicyclohexylcarbodiimid in wenig DMF zugetropft. Nach vollstaenediger Bildung des aktivierten Esters wird 5,7 g (30 mmol) 5,6-Diamino-3,3-dimethyl-indolin-2-on zugefuegt und 30 Minuten nachgeruehrt. Das Dimethylformamid wird im Hochvakuum abdestilliert, der Rueckstand mit Wasser durchgearbeitet und das Rohprodukt aus Monoamid und Dicyclohexylharnstoff abgesaugt und ohne Reinigung weiterverarbeitet.

Ausbeute: 20 g.

b) Der nach a) erhlatene Rueckstand wird mit 200 ml Ethanol und 40 ml conc. Salzsaeure versetzt und 2 Stunden zum Rueckfluß erhitzt. Nach dem Abkuehlen wird der Dicyclohexylharnstoff abgesaugt und die ethanolische Loesung im Vakuum eingeengt. Der Rueckstand wird in Wasser suspendiert, mit 2 N Ammoniak alkalisch gestellt, die Titelverbindung abgesaugt und unter Kuehlung aus Essigester umkrisallisiert.

Ausbeute: 1.0 g (12% d.Th.); Mp. >360.

Beispiel 7

7-Methyl-2-(4-pyridazinyl)-6,7-dihydro-3H,5H-pyrrolo[2,3-f]benzimidazol-6-on

Analog Beispiel 6 wurde ausgehend von 6.0 g (24 mmol) 5,6-Diamino-3-methyl-indolin-2-on-dihydrochlorid und 3.6 g (28.8 mmol) Pyridazin-4-carbonsaeure die Titelverbindung erhalten. Die Reinigung erfolgte durch Saeulechromatographie an Kieselgel (Laufmittel: Methylenchlorid/Methanol 9:1).

Ausbeute: 0.15 g (2.4% d.Th.); Mp. >340°C.

Beispiel 8

7,7-Dimethyl-2-(5-pyrimidinyl)-6,7-dihydro-3H,5H-pyrrolo[2,3-f]benzimidazol-6-on

Analog zu Beispiel 6 wurden aus 0.164 g (1.32 mmol) Pyrimidin-5-carbonsaeure und 0.191 g (1 mmol) 5,6-Diamino-3,3-dimethylindolin-2-on nach Cyclisierung des Rohprodukts mit Eisessig die Titelverbindung erhalten.

Ausbeute: 70 mg (25% d.Th.); Mp. 310—12°C aus Dioxan.

Beispiel 9

7,7-Dimethyl-2-(4-pyrimidinyl)-6,7-dihydro-3H,5H-pyrrolo[2,3-f]benzimidazol-6-on

Analog zu Beispiel 2 erhaelt man aus 60 mg (0.55 mmol) Pyrimidin-4-aldehyd und 0.103 g (0.54 mmol) 5,6-Diamino-3,3-dimethyl-indolin-2-on nach Saeulenchromatographie an Kieselgel (Laufmittel: Methylenchlorid/Methanol 9:1) die Titelverbindung.

Ausbeute: 10 mg (6.6% d.Th.); Mp. >300°C.

Beispiel 10

7,7-Dimethyl-2-(2-methyl-pyrimidin-5-yl)-6,7-dihydro-3H,5H-pyrrolo[2,3-f]benzimidazol-6-on

Analog zu Beispiel 6 wurde ausgehend von 5.7 g (30 mmol) 5,6-Diamino-3,3-dimethyl-indolin-2-on und 4.8 g (35 mmol) 2-Methyl-pyrimidin-5-carbonsaeure das Monoamid erhalten.

Ausbeute: 3.6 g (39% d.Th.); Mp. 166—70°C aus Wasser.

Das Rohprodukt wurd anschließend mit Eisessig zur obigen Titelverbindung cyclisier.

Ausbeute: 2.3 g (68.5% d.Th); Mp >350°C.

Beispiel 11

7,7-Dimethyl-2-(4-imidazolyl)-6,7-dihydro-3H,5H-pyrrolo[2,3-f]benzimidazol-6-on

Analog zu Beispiel 4 erhaelt man ausgehend von 2.7 g (14.1 mmol) 5,6-Diamino-3,3-dimethyl-indolin-2-on und 2.8 g (21.5 mmol) Imidazol-4-carbonsaeurechlorid die Titelverbindung.

Ausbeute: 0.17 g (4.5% d.Th.); Mp. 270°C aus Methanol.

Beispiel 12

7,7-Dimethyl-2-(6-hydroxy-pyridazin-3-yl)-6,7-dihydro-3H,5H-pyrrolo[2,3-f]benzimidazol-6-on

Analog zu Beispiel 6 erhaelt man ausgehend von 4.9 g (35 mmol) 6-Hydroxy-pyridazin-3-carbonsaeure und 5.7 g (30 mmol) 5,6-Diamino-3,3-dimethyl-indolin-2-on die Titelverbindung.

Ausbeute: 2.8 g (32% d.Th.); Mp. >350°C aus Ethanol.

Beispiel 13

7,7-Dimethyl-2-(1,2,4-1H-triazol-3-yl)-6,7-dihydro-3H,5H-pyrrolo[2,3-f]benzimidazol-6-on

Analog Beispiel 2 erhaelt man ausgehend von 0.38 g (2 mmol) 5,6-Diamino-3,3-dimethyl-indolin-2-on und 0.195 g (2 mmol) 1,2,4-Triazol-3-aldehyd die Titelverbindung.

Ausbeute: 0.15 g (28% d.Th.); Mp. >350°C.

Beispiel 14

7,7-Dimethyl-2-(2-methyl-oxazol-4-yl)-6,7-dihydro-3H,5H-pyrrolo[2,3-f]benzimidazol-6-on

Analog zu Beispiel 10 erhaelt man ausgehend von 764 mg (4 mmol) 5,6-Diamino-3,3-dimethyl-indolin-2-on und 672 mg (5.32 mmol) 2-Methyl-oxazol-4-carbonsaeure nach Reinigung an Kieselgel (Laufmittel: Methylenchlorid/Methanol 8:2) die Titelverbindung.

Ausbeute: 0.3 g (26.6% d.Th.); Mp. 315—18°C.

Beispiel 15

7,7-Dimethyl-2-(5-methyl-pyrazol-3-yl)-6,7-dihydro-3H,5H-pyrrolo[2,3-f]benzimidazol-6-on

Analog zu Beispiel 2 erhaelt man ausgehend von 0.57 g (3 mmol) 5,6-Diamino-3,3-dimethyl-indolin-2-on und 0.33 g (3 mmol) 5-Methyl-pyrazol-3-aldehyd nach alkalisch stellen des Ansatzes mit konzentriertem Ammoniak das Rohprodukt der Titelverbindung. Der Rueckstand wurde ueber Kieselgel (Laufmittel: Methylenchlorid/$NH_3$ gesaettigtes Methanol 10:3) gereinigt.

Ausbeute: 0.22 g (26% d.Th.); Mp. 245—50 aus Wasser.

Beispiel 16

7-Methyl-7-ethoxycarbonyl-2-(2-pyrazinyl)-6,7-dihydro-3H,5H-pyrrolo[2,3-f]benzimidazol-6-on

a) 5.0 g (18.5 mmol) 3-Methyl-3-ethoxycarbonyl-6-amino-indolin-2-on-hydrochlorid wurde in 60 ml Methylenchlorid suspendiert und mit 12.5 ml (90.4 mmol) Triethylamin versetzt. Unter Eiskuehlung wurden dann 6.4 g (45.2 mmol) Pyrazin-2-carbonsaeurechlorid portionsweise zugesetzt und drei Stunden weitergeruehrt. Der Ansatz wurde eingeengt, mit Wasser durchgearbeitet, abgesaugt und durch Saeulenchromatographie (Laufmittel: Methylenchlorid/$CH_3OH$ 99:1) an Kieselgel gereingt.

Man erhaelt 4.0 g (65%) der Titelverbindung vom Mp.

b) 3.4 g (10 mmol) 3-Methyl-3-ethoxycarbonyl-6-(2-pyrazinoylamino)-indolin-2-on wurden in 20 ml konzentrierter Schwefelsaeure geloest und bei 0—5°C mit einer Loesung von 1.1 g (11 mmol) Kaliumnitrat in konzentrierter Schwefelsaeure tropfenweise versetzt. Nach 3 Stunden wurde auf Eis gegossen, abgesaugt, der Rueckstand in Wasser suspendiert, mit waessrigem Ammoniak neutralisiert, abgesaugt und aus Ethanol umkristallisiert. Man erhaelt 3.2 g (83%) 3-Methyl-3-ethoxycarbonyl-5-nitro-6-(2-pyrazinoylamino)indolin-2-on vom Mp.

c) 3.2 g (8.3 mmol) 3-Methyl-3-ethoxycarbonyl-5-nitro-6-(2-pyrazinoylamino-indolin-2-on in 100 ml Ethanol wurden mit 0.5 g 10 proz. Palladium auf Kohle hydriert. Nach beendeter Wasserstoffaufnahme wurde die Loesung von Katalysator abgesaugt, das Ethanol abgedampft und der Rueckstand 1 Stunde in Eisessig bei 60°C geruehrt. Anschließend wurde der Eisessig abdestilliert, der Rueckstand in Wasser aufgenommen, neutralasiert, abgesaugt und aus Ethanol umkristallisiert. Man erhaelt 1.8 g (64%) der Titelverbindung vom Mp.

Beispiel 17

7,7-Dimethyl-2-(1,2,5-thiadiazol-3-yl)-6,7-dihydro-3H,5H-pyrrolo[2,3-f]benzimidazol-6-on

2.0 g (15.3 mmol) 1,2,5-Thiadiazol-3-carbonsaeure und 2.6 g (13.6 mmol) 5,6-Diamino-3,3-dimethyl-indolin-2-on wurden in 40 ml Polyphosphorsaeure unter Stickstoff 2 Stunden auf 160°C erhitzt. Die warme Loesung wurde auf Eiswasser gegossen, durchgearbeitet und die Kristalle abgesaugt. Der Rueckstand wurd nochmals in Wasser suspendiert, mit waessrigem Ammoniak neutralisiert, abgesaugt und aus Methanol unter Zusatz von Kohle umkristallisiert.

Ausbeute: 2.6 g (66% d.Th.); Mp. >300°C.

**Patentansprüche**

1. Pyrrolo-Benzimidazole der Formel I

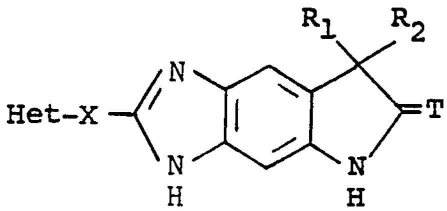

(I)

in welcher

$R_1$ ein Wasserstoffatom, eine $C_1$—$C_6$-Alkyl-, $C_2$—$C_6$-Alkenyl- oder $C_3$—$C_7$-Cycloalkylgruppe bedeutet,

$R_2$ ein Wasserstoffatom, eine $C_1$—$C_6$-Alkyl-, $C_2$—$C_6$-Alkenyl- oder Cyanogruppe, eine durch eine Hydroxy-, $C_1$—$C_6$-Alkyl, $C_1$—$C_6$-Alkoxy-, Amino-, $C_1$—$C_6$-Alkylamino-, Di-$C_1$—$C_6$-alkylamio- oder Hydrazinogruppe substituierte Carbonylgruppe oder mit $R_1$ zusammen eine $C_3$—$C_8$-Cycloalkylengruppe darstellt oder $R_1$ und $R_2$ zusammen eine Alkyliden- oder Cycloalkylidengruppe bilden,

X einen Valenzstrich, eine $C_1$—$C_4$-Alkylengruppe oder die Vinylengruppe bedeutet,

T ein Sauerstoff- oder Schwefelatom ist, und

Het einen Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- oder Tetrazinylrest darstellt und diese Reste durch eine oder mehrere $C_1$—$C_6$-Alkyl-, $C_1$—$C_6$-Alkoxy-, $C_1$—$C_6$-Alkyl-mercapto, Hydroxy-, Nitro-, Amino-, Halogen- oder Cyan-gruppen substituiert sein können, deren Tautomere und deren physiologisch verträgliche Salze anorganischer und organischer Säuren.

2. Pyrrolo-Benzimidazole der Formel I gemäß Anspruch 1, in welcher

$R_1$ Wasserstoff, eine Methyl-, Ethyl-, Cyclopentyl- oder Cyclohexyl-Gruppe bedeutet,

$R_2$ Wasserstoff, eine Methyl-, Ethyl- Isopropyl-, 3-Pentyl-, Allyl-, Cyan, Acetyl-, Propionyl-, Methoxycarbonyl, Ethoxycarbonyl-, Aminocarbonyl-, Methylaminocarbonyl-, Dimethylaminocarbonyl- und die Hydrazinocarbonyl-Gruppe bedeutet, oder

$R_1$ und $R_2$ zusammen mit dem C-Atom, an das sie gebunden sind, einen Spirocyclopropyl-, Spirocyclobutyl-, Spirocyclopentyl- und Spirocyclohexylring darstellen oder

$R_1$ und $R_2$ zusammen eine Isopropylidengruppe darstellen,

X ein Valenzstrich, eine Methylen- oder Vinylengruppe bedeuten,

T für ein Sauerstoffatom steht, und

Het einen Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- und der Tetrazinylrest bedeuten, wobei die Reste Het ein- oder mehrfach durch Hydroxy, Methyl, Methoxy, Methylmercapto, Amino, Nitro, Halogen oder Cyano substituiert sein können.

3. Pyrrolo-Benzimidazole der Formel I gemäß Anspruch 1 oder 2, in welcher

$R_1$ Wasserstoff, Methyl oder Ethyl,

$R_2$ Methyl, Ethyl oder Ethoxycarbonyl oder

$R_1$ und $R_2$ zusammen mit dem C-Atom, an das sie gebunden sind, einen Spirocyclopentan-Ring darstellen,

X eine Velenzstrisch oder eine —$CH_2$-Gruppe bedeuten,

T Sauerstoff und

Het einen Furanyl-, Thienyl- Pyrrolyl-, Thiazolyl-, Imidazolyl-, Triazolyl-, Oxazolyl-, Pyrazolyl-, Thiadiazolyl-, Pyrazinyl-, Pyridazinyl- oder Pyrimidinyl-Ring darstellt, wobei die Heterocyclen ein- oder mehrfach durch Hydroxy oder Methyl substituiert sein können.

4. Pyrrolo-Benzimidazole der Formel I gemäß Anspruch 1, 2 oder 3, in welcher

$R_1$ und $R_2$ die Methyl- oder Ethylgruppe bedeuten,

X ein Valenzstrich,

T Sauerstoff und

Het einen Furanyl-, Thienyl- Pyrrolyl-, Pyrazinyl-, Thiazolyl-, Pyridazinyl- oder Pyrimidinyl-Ring darstellt.

5. Verfahren zur Herstellung von Pyrrolo-Benzimidazolen der Formel I

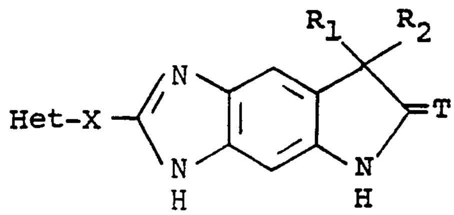

(I)

in welcher

$R_1$ ein Wasserstoffatom, eine $C_1$—$C_6$-Alkyl-, $C_2$—$C_6$-Alkenyl- oder $C_3$—$C_7$-Cycloalkylgruppe bedeutet,

$R_2$ ein Wasserstoffatom, eine $C_1$—$C_6$-Alkyl-, $C_2$—$C_6$-Alkenyl- oder Cyanogruppe, eine durch eine Hydroxy-, $C_1$—$C_6$-Alkyl, $C_1$—$C_6$-Alkoxy-, Amino-, $C_1$—$C_6$-Alkylamino-, Di-$C_1$—$C_6$-alkylamio- oder Hydrazinogruppe substituierte Carbonylgruppe oder mit $R_1$ zusammen eine $C_3$—$C_8$-Cycloalkylengruppe darstellt oder $R_1$ und $R_2$ zusammen eine Alkyliden- oder Cycloalkylidengruppe bilden,

X einen Valenzstrich, eine $C_1$—$C_4$-Alkylengruppe oder die Vinylengruppe bedeutet,

T ein Sauerstoff- oder Schwefelatom ist, und

Het einen Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- oder Tetrazinylrest darstellt und diese Reste durch eine oder mehrere $C_1$—$C_6$-Alkyl-, $C_1$—$C_6$-Alkoxy-, $C_1$—$C_6$-Alkylmercapto, Hydroxy-, Nitro-, Amino-, Halogen- oder Cyan-gruppen substituiert sein können, deren Tautomere und deren physiologisch verträgliche Salze anorganischer und organischer Säuren, dadurch gekennzeichnet, daß man in an sich bekannter Weise

a) eine Verbindung der Formel VI

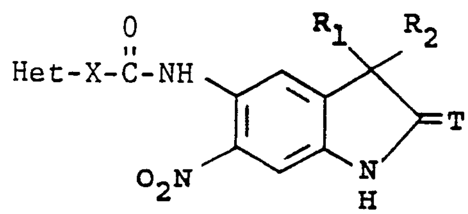

(VI)

oder der Formel VII

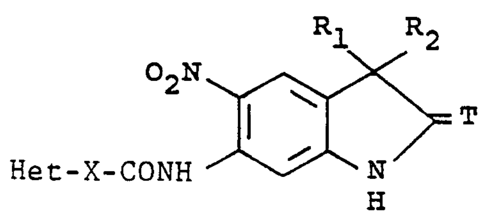

(VII)

in denen $R_1$, $R_2$, T, X und Het die oben angegebenen Bedeutungen haben, reduziert und anschließend cyclisiert oder

b) eine Verbindung der Formel VIII

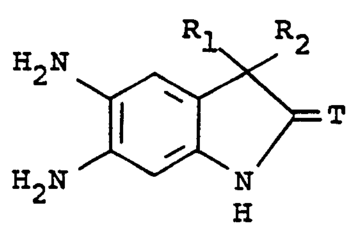

(VIII)

in denen $R_1$, $R_2$ und T die oben angegebenen Bedeutungen haben, mit einer Verbindung der Formel IX

$$Het—X—CO—Y \quad (IX)$$

in der Het und X die oben angegebenen Bedeutungen haben und Y Wasserstoff oder einen reaktiven Rest

darstellt, umsetzt und anschließend cyclisiert oder

c) eine Verbindung der Formel X

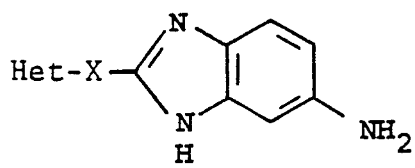

(X)

mit entsprechenden Ketonen und Natriumbisulfit oder entsprechenden Carbonsäurehydraziden oder Carbonsäureamiden zum Oxindol cyclisiert,

anschließend gewünschtenfalls erhaltene Verbindungen der Formel I in andere Verbindugen der Formel I überführt, sowie gewünschtenfalls die Verbindungen mit anorganischen oder organischen Säuren in physiologisch verträgliche Salze überführt.

6. Verfahren gemäß Anspruch 5 zur Herstellung von Verbindungen der Formel I, in welcher

$R_1$ Wasserstoff, eine Methyl-, Ethyl-, Cyclopentyl- oder Cyclohexylgruppe bedeutet,

$R_2$ Wasserstoff, eine Methyl-, Ethyl-, Isopropyl-, 3-Pentyl-, Allyl-, Cyan-, Acetyl-, Propionyl-, Methoxycarbonyl- Ethoxycarbonyl, Aminocarbonyl-, Methylaminocarbonyl-, Dimethylaminocarbonyl- und die Hydrazinocarbonyl-Gruppe bedeutet, oder

$R_1$ und $R_2$ zusammen mit dem C-Atom, an das sie gebunden sind, einen Spirocyclopropyl-, Spirocyclobutyl-, Spirocyclopentyl- und Spirocyclohexylring darstellen oder

$R_1$ und $R_2$ zusammen eine Isopropylidengruppe darstellen,

X ein Valenzstrich, eine Methylen- oder Vinylengruppe bedeuteten,

T für ein Sauerstoffatom steht, und

Het einen Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- und der Tetrazinylrest bedeuten, wobei die Reste Het ein- oder mehrfach durch Hydroxy, Methyl, Methoxy, Methylmercapto, Amino, Nitro, Halogen oder Cyano substituiert sein können.

7. Verfahren gemäß Anspruch 5 oder 6 zur Herstellung von Verbindungen der Formel I, in welcher

$R_1$ Wasserstoff, Methyl oder Ethyl,

$R_2$ Methyl, Ethyl oder Ethoxycarbonyl oder

$R_1$ und $R_2$ zusammen mit dem C-Atom, an das sie gebunden sind, einen Spirocyclopentan-Ring darstellen,

X einen Valenzstrich oder eine $—CH_2—$Gruppe bedeutet,

T Sauerstoff und

Het einen Furanyl-, Thienyl, Pyrrolyl-, Thiazolyl-, Imidazolyl-, Triazolyl-, Oxazolyl-, Pyrazolyl-, Thiadiazolyl-, Pyrazinyl-, Pyridazinyl- oder Pyrimidinyl-Ring darstellt, wobei die Heterocyclen ein- oder mehrfach durch Hydroxy oder Methyl substituiert sein können.

8. Verfahren gemäß Anspruch 5, 6 oder 7 zur Herstellung von Verbindungen der Formel I, in welcher

$R_1$ und $R_2$ die Methyl- oder Ethylgruppe bedeuten,

X ein Valenzstrich bedeutet,

T Sauerstoff und

Het eine Furanyl-, Thienyl, Pyrrolyl-, Pyrazinyl-, Thiazolyl-, Pyridazinyl- oder Pyrimidinyl-Ring darstellt.

9. Arzneimittel, enthaltend eine Verbindung gemäß Anspruch 1, 2, 3 oder 4 neben üblichen Träger- und Hilfsstoffen.

10. Verwendung von Verbindungen gemäß Anspruch 1, 2, 3 oder 4 zur Herstellung von Arzneimitteln zur Prophylaxe bzw. Behandlung von Herz- und Kreislauferkrankungen.

11. Verbindungen der Formel VI

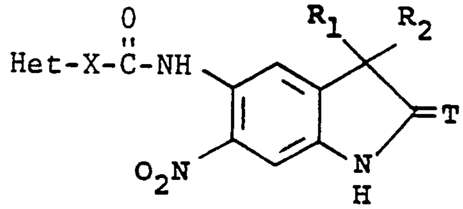

(VI)

in der $R_1$, $R_2$, X, T und Het die in Anspruch 1 angegebenen Bedeutungen haben, deren Tautomere und deren physiologisch verträgliche Salze anorganischer und organischer Säuren.

12. Verbindungen der Formel VII

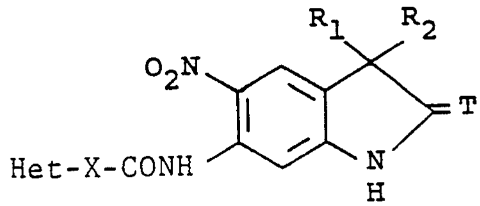

(VII)

in welcher $R_1$, $R_2$, X, T und Het die in Anspruch 1 angegebenen Bedeutungen haben, deren Tautomere und deren physiologisch verträgliche Salze anorganischer und organischer Säuren.

**Revendications**

1. Pyrrolo-benzimidazoles de formule I

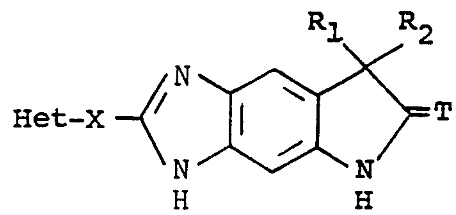

(I)

où

$R_1$ représente un atome d'hydrogène, un groupe alkyle en $C_1$—$C_6$, un groupe alcényle en $C_2$—$C_6$ ou un groupe cycloalkyle en $C_3$—$C_7$,

$R_2$ représente un atome d'hydrogène, un groupe alkyle en $C_1$—$C_6$, un groupe alcényle en $C_2$—$C_6$ ou un groupe cyano, un groupe carbonyle substitué par un groupe hydroxy, un groupe alkyle en $C_1$—$C_6$, alcoxy en $C_1$—$C_6$, amino, alkyle($C_1$—$C_6$)-amino, dialkyl($C_1$—$C_6$)-amino ou hydrazino, ou conjointement avec $R_1$, un groupe cycloalkylène($C_3$—$C_8$), ou $R_1$ et $R_2$ représentent conjointement un groupe alkylidène ou cycloalkylidène,

X représente une valence libre, un groupe alkylène en $C_1$—$C_4$ ou un groupe vinylène,

T représente un atome d'oxygène ou de soufre, et

Het représente un groupe pyrrolyle, furanyle, thiophényle, pyrazolyle, imidazolyle, thiazolyle, oxazolyle, triazolyle, tétrazolyle, thiadiazolyle, oxadiazolyle, pyrazinyle, N,N-dioxypyrazinyle, pyrimidinyle, N,N-dioxypyrimidinyle, pyridazinyle, oxazinyle, thiazinyle, triazinyle ou tétrazinyle, et ce groupe peut être substitué par un ou plusieurs groupes alkyle en $C_1$—$C_6$, alcoxy en $C_1$—$C_6$, alkyle($C_1$—$C_6$)-mercapto, hydroxy, nitro, amino, halogène ou cyano, leurs tautomères et leurs sels minéraux et organiques physiologiquement acceptables.

2. Pyrrolo-benzimidazoles selon la revendication 1, dans lesquels

$R_1$ représente un atom d'hydrogène, un groupe méthyle, éthyle, cyclopentyle ou cyclohexyle,

$R_2$ représente un atome d'hydrogène, un groupe méthyle, éthyle, isopropyle, 3-pentyle, allyle, cyano, acétyle, propionyle, méthoxycarbonyle, éthoxycarbonyle, aminocarbonyle, méthylaminocarbonyle, diméthylaminocarbonyle et hydrazinocarbonyle, ou

$R_1$ et $R_2$ représentent, conjointement avec l'atome de C auquel ils sont liés, un cycle spirocyclopropyle, spirocyclobutyle, spirocyclopentyle et spirocyclohexyle, ou

$R_1$ et $R_2$ représentent conjointement un groupe isopropylidène,

X représente une valence libre, un groupe méthylène ou vinylidène,

T représente un atome d'oxygène, et

Het représente un groupe pyrrolyle, furanyle, thiophényle, pyrazolyle, imidazolyle, thiazolyle, oxazolyle, triazolyle, tétrazolyle, thiadiazolyle, oxadiazolyle, pyrazinyle, N,N-dioxypyrazinyle, pyrimidinyle, N,N-dioxypyrimidinyle, pyridazinyle, oxazinyle, thiazinyle, triazinyle ou tétrazinyle,

les groupes Het pouvant être substitués une ou plusieurs fois par un groupe hydroxy, méthyle, méthoxy, méthylmercapto, amino, nitro, halogène ou cyano.

3. Pyrrolo-benzimidazoles selon la revendication 1 ou 2, dans lequels

$R_1$ représente un atom d'hydrogène, un groupe méthyle ou éthyle,

$R_2$ un un groupe méthyle, éthyle ou éthoxycarbonyle, ou

$R_1$ et $R_2$ représentent, conjointement avec l'atome de C auquel ils sont liés, un cycle spirocyclopentane,

X représente une valence libre ou un groupe $—CH_2$,

T représente un atome d'oxygène, et

Het représente un groupe furanyle, thiényle, pyrroyle, thiazolyle, imidazolyle, triazolyle, oxazolyle, pyrazolyle, thiadiazolyle, pyrazinyle, pyridazinyle ou pyrimidinyle, les hétérocycles pouvant être substitués une ou plusieurs fois par un groupe hydroxy ou méthyle.

4. Pyrrolo-benzimidazoles selon la revendication 1, 2 ou 3, dans lesquels

$R_1$ et $R_2$ représentent le groupe méthyle ou éthyle,

X représente une valence libre,

T représente un atome d'oxygène, et

Het représente un groupe furanyle, thiényle, pyrrolyle, pyrazinyle, thiazolyle, pyridazinyle ou pyrimidinyle.

5. Procédé pour la préparation de pyrrolo-benzimidazoles de formule I

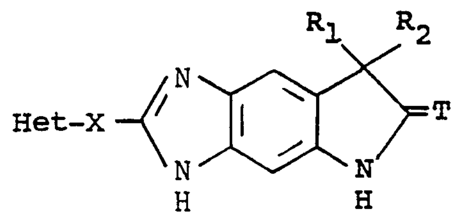

(I)

où

$R_1$ représente un atome d'hydrogène, un groupe alkyle en $C_1$—$C_6$, un groupe alcényle en $C_2$—$C_6$ ou un groupe cycloalkyle en $C_3$—$C_7$,

$R_2$ représente un atome d'hydrogène, un groupe alkyle en $C_1$—$C_6$, un groupe alcényle en $C_2$—$C_6$ ou un groupe cyano, un groupe carbonyle substitué par un groupe hydroxy, un groupe alkyle en $C_1$—$C_6$, alcoxy en $C_1$—$C_6$, amino, alkyle($C_1$—$C_6$)-amino, dialkyl($C_1$—$C_6$)-amino ou hydrazino, ou conjointement avec $R_1$, un groupe cycloalkylène($C_3$—$C_8$), ou $R_1$ et $R_2$ représentent conjointement un groupe alkylidène ou cycloalkylidène,

X représente une valence libre, un groupe alkylène en $C_1$—$C_4$ ou un groupe vinylène,

T représente un atome d'oxygène ou de soufre, et

Het représente un groupe pyrrolyle, furanyle, thiophényle, pyrazolyle, imidazolyle, thiazolyle, oxazolyle, triazolyle, tétrazolyle, thiadiazolyle, oxadiazolyle, pyrazinyle, N,N-dioxypyrazinyle, pyrimidinyle, N,N-dioxypyrimidinyle, pyridazinyle, oxazinyle, thiazinyle, triazinyle ou tétrazinyle, et ce groupe peut être substitué par un ou plusieurs groupes alkyle en $C_1$—$C_6$, alcoxy en $C_1$—$C_6$, alkyle($C_1$—$C_6$)-mercapto, hydroxy, nitro, amino, halogène ou cyano, leurs tautomères et leurs sels minéraux et organiques physiologiquement acceptables, caractérisé en ce que, de manière connue en soi,

a) on réduit un composé de formule VI

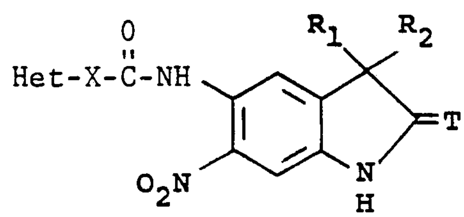

(VI)

ou de formule VII

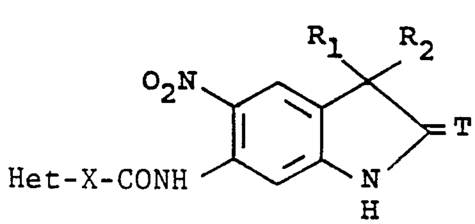

(VII)

où $R_1$, $R_2$, T, X et Het ont les significations mentionnées ci-dessus, et ensuite, on le cyclise, ou

b) on fait réagir un composé de formule VIII

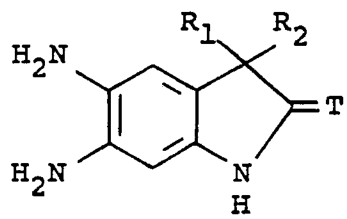

(VIII)

où $R_1$, $R_2$ et T ont les significations mentionnées ci-dessus, avec un composé de formule IX

Het—X—CO—Y (IX)

où Het et X ont les significations mentionnées ci-dessus et Y représente un atome d'hydrogène ou un groupe réactif, en ensuite, on le cyclise, ou

c) on cyclise un composé de formule X

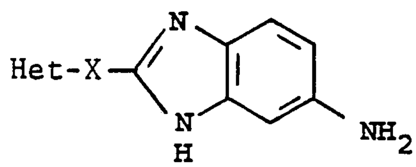

(X)

avec des cétones appropriées et du bisulfite de sodium ou des hydrazides d'acides carboxyliques appropriés ou des amides d'acides carboxyliques appropriés, en oxindole, ensuite, on transforme éventuellement les composés de formule I obtenus en d'autres composés de formule I, de même qu'éventuellement, on transforme les composés, à l'aide d'acides minéraux ou organiques, en sels physiologiquement acceptables.

6. Procédé selon la revendication 5, pour la préparation de composés de formule I, dans lesquels

$R_1$ représente un atome d'hydrogène, un groupe méthyle, éthyle, cyclopentyle ou cyclohexyle,

$R_2$ représente un atome d'hydrogène, un groupe méthyle, éthyle, isopropyle, 3-pentyle, allyle, cyano, acétyle, propionyle, méthoxycarbonyle, éthoxycarbonyle, aminocarbonyle, méthylaminocarbonyle, diméthylaminocarbonyle et hydrazinocarbonyle, ou

$R_1$ et $R_2$ représentent, conjointement avec l'atome de C auquel ils sont liés, un cycle spirocyclopropyle, spirocyclobutyle, spirocyclopentyle et spirocyclohexyle, ou

$R_1$ et $R_2$ représentent conjointement un groupe isopropylidène,

X représente une valence libre, un groupe méthylène ou vinylidène,

T représente un atome d'oxygène, et

Het représente un groupe pyrrolyle, furanyle, thiophényle, pyrazolyle, imidazolyle, thiazolyle, oxazolyle, triazolyle, tétrazolyle, thiadiazolyle, oxadiazolyle, pyrazinyle, N,N-dioxypyrazinyle, pyrimidinyle, N,N-dioxypyrimidinyle, pyridazinyle, oxazinyle, thiazinyle, triazinyle ou tétrazinyle,

les groupes Het pouvant être substitués une ou plusieurs fois par un groupe hydroxy, méthyle, méthoxy, méthylmercapto, amino, nitro, halogène ou cyano.

7. Procédé selon la revendication 5 ou 6, pour la préparation de composés de formule I, dans lesquels

$R_1$ représente un atom d'hydrogène, un groupe méthyle ou éthyle,

$R_2$ un groupe méthyle, éthyle ou éthoxycarbonyle, ou

$R_1$ et $R_2$ représentent, conjointement avec l'atome de C auquel ils sont liés, un cycle spirocyclopentane,

X représente une valence libre ou un groupe $—CH_2$,

T représente un atome d'oxygène, et

Het représente un groupe furanyle, thiényle, pyrroyle, thiazolyle, imidazolyle, triazolyle, oxazolyle, pyrazolyle, thiadiazolyle, pyrazinyle, pyridazinyle ou pyrimidinyle, les hétérocycles pouvant être substitués une ou plusieurs fois par un groupe hydroxy ou méthyle.

8. Procédé selon la revendication 5, 6 ou 7, pour la préparation de composés de formule I, dans lesquels

$R_1$ et $R_2$ représentent le groupe méthyle ou éthyle,

X représente une valence libre,

T représente un atome d'oxygène, et

Het représente un groupe furanyle, thiényle, pyrrolyle, pyrazinyle, thiazolyle, pyridazinyle ou pyrimidinyle.

9. Médicament contenant un composé selon la revendication 1, 2, 3 ou 4, en plus des véhicules et adjuvants usuels.

10. Utilisation des composés selon la revendication 1, 2, 3 ou 4, pour la préparation de médicaments pour la prophylaxie ou le traitement des maladies cardiovasculaires.

11. Composés de formule VI

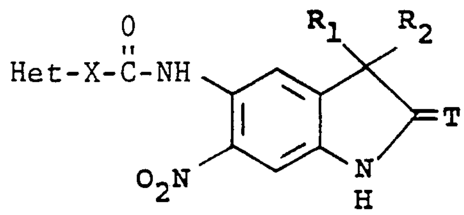

(VI)

où $R_1$, $R_2$, T, X et Het ont les significations mentionnées dans la revendication 1, leurs tautomères et leurs sels d'acides minéraux et organiques physiologiquement acceptables.

12. Composés de formule VII

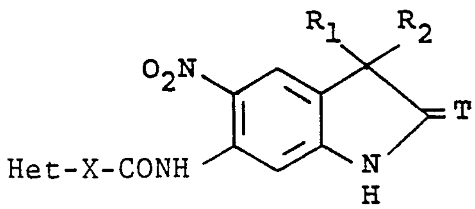

(VII)

où $R_1$, $R_2$, T, X et Het ont les significations mentionnées dans la revendication 1, leurs tautomères et leurs sels d'acides minéraux et organiques physiologiquement acceptables.

**Claims**

1. Pyrrolobenzimidazoles of the formula I

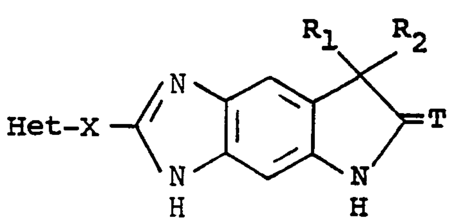

(I)

in which

$R_1$ signifies a hydrogen atom, a $C_1$—$C_6$-alkyl, $C_2$—$C_6$-alkenyl or $C_3$—$C_7$-cycloalkyl group,

$R_2$ a hydrogen atom, a $C_1$—$C_6$-alkyl, $C_2$—$C_6$-alkenyl or cyano group, a carbonyl group substituted by a hydroxyl, $C_1$—$C_6$-alkyl-, $C_1$—$C_6$-alkoxy, amino, $C_1$—$C_6$-alkylamino, di-$C_1$—$C_6$-alklyamino or hydrazino group, or together with $R_1$, represents a $C_3$—$C_8$-cycloalkylidene group, or

$R_1$ and $R_2$ together form an alkylidene or cycloalkylidene group,

X signifies a valency bond, a $C_1$—$C_4$-alkylene group or the vinylene group,

T is an oxygen or sulphur atom and

Het represents a pyrrolyl, furanyl, thiophenyl, pyrazolyl, imidazolyl, thiazolyl, oxazolyl, triazolyl, tetrazolyl, thiadiazolyl, oxadiazolyl, pyrazinyl, N,N-dioxypyrazinyl, pyrimidinyl, N,N-dioxypyrimidinyl, pyridazinyl, oxazinyl, thiazinyl, triazinly or tetrazinyl radical and these radicals can be substituted by one or more $C_1$—$C_6$-alkyl, $C_1$—$C_6$-alkoxy, $C_1$—$C_6$-alkylmercapto, hydroxyl, nitro, amino, halogeno or cyano groups; their tautomers and their physiologically acceptable salts of inorganic and organic acids.

2. Pyrrolobenzimidazoles of the formula I according to claim 1, in which

$R_1$ signifies hydrogen, a methyl, ethyl, cyclopentyl or cyclohexyl group,

$R_2$ hydrogen, a methyl, ethyl, isopropyl, 3-pentyl, allyl, cyano, acetyl, propionyl, methoxycarbonyl, ethoxycarbonyl, aminocarbonyl, methylaminocarbonyl, dimethylaminocarbonyl and the hydrazinocarbonyl group or

$R_1$ and $R_2$, together with the C-atom to which they are attached, represent a spirocyclopropyl, spiorcyclobutyl, spirocyclopentyl and spirocyclohexyl ring or $R_1$ and $R_2$ together represent an isopropylidene group,

X signifies a valency bond, a methylene or vinylene group,

T stands for an oxygen atom and

Het signifies a pyrrolyl, furanyl, thiophenyl, pyrazolyl, imidazolyl, thiazolyl, oxazolyl, triazolyl, tetrazolyl, thiadiazolyl, oxadiazolyl, pyrazinyl, N,N-dioxypyrazinyl, pyrimidinyl, N,N-dioxypyrimidinyl, pyridazinyl, oxazinyl, thiazinyl, triazinyl and the tetrazinyl residue, whereby the radicals Het can be substituted one or more times by hydroxyl, methyl, methoxy, methylmercapto, amino, nitro, halogeno or cyano.

3. Pyrrolobenzimidazoles of the formula I according to claim 1 or 2, in which

$R_1$ signifies hydrogen, methyl, or ethyl,

$R_2$ methyl, ethyl or ethoxycarbonyl or

$R_1$ and $R_2$, together with the C-atom to which they are attached, a spirocyclopentane ring,

X signifies a valency bond or a $—CH_2$-group,

T oxygen atom and

Het is a furanyl, thienyl, pyrrolyl, thiazolyl, imidazolyl, triazolyl, oxazolyl, pyrazolyl, thiazolyl, imidazolyl, triazolyl, oxazolyl, pyrazolyl, thiadiazolyl, pyrazinyl, pyridazinyl or pyrimidinyl ring, whereby the heterocycles can be substituted one or more times by hydroxyl or methyl.

4. Pyrrolobenzimidazoles of the formula I according to claim 1, 2 or 3, in which

$R_1$ and $R_2$ signify the methyl or ethyl group,

X a valency bond,

T oxygen and

Het a furanyl, thienyl, pyrrolyl, pyrazinyl, thiazolyl, pyridazinyl or pyrimidinyl ring.

5. Process for the preparation of pyrrolobenzimidazoles of the formula I

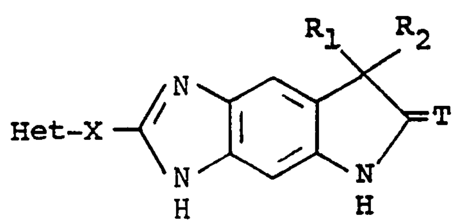

(I)

in which

$R_1$ signifies a hydrogen atom, a $C_1$—$C_6$-alkyl, $C_2$—$C_6$-alkenyl or $C_3$—$C_7$-cycloalkyl group,

$R_2$ represents a hydrogen atom, a $C_1$—$C_6$-alkyl, $C_2$—$C_6$-alkenyl or cyano group, a carbonyl group substituted by a hydroxyl, $C_1$—$C_6$-alkyl-, $C_1$—$C_6$-alkoxy, amino, $C_1$—$C_6$-alkylamino, di-$C_1$—$C_6$-alklyamino or hydrazino group, or together with $R_1$, represents a $C_3$—$C_8$-cycloalkylidene group, or

$R_1$ and $R_2$ together form an alkylidene or cycloalkylidene group,

X signifies a valency bond, a $C_1$—$C_4$-alkylene group or the vinylene group,

T is an oxygen or sulphur atom and

Het represents a pyrrolyl, furanyl, thiophenyl, pyrazolyl, imidazolyl, thiazolyl, oxazolyl, triazolyl, tetrazolyl, thiadiazolyl, oxadiazolyl, pyrazinyl, N,N-dioxypyrazinyl, pyrimidinyl, N,N-dioxypyrimidinyl, pyridazinyl, oxazinyl, thiazinyl, triazinly or tetrazinyl radical and these radicals can be substituted by one or more $C_1$—$C_6$-alkyl, $C_1$—$C_6$-alkoxy, $C_1$—$C_6$-alkylmercapto, hydroxyl, nitro, amino, halogeno or cyano groups; of their tautomers and of their physiologically acceptable salts of inorganic and organic acids, characterised in that, in per se known manner, one

a) reduces a compound of the formula VI

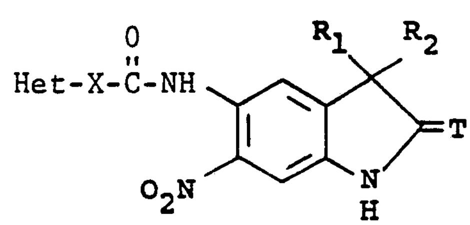

(VI)

or of the formula VII

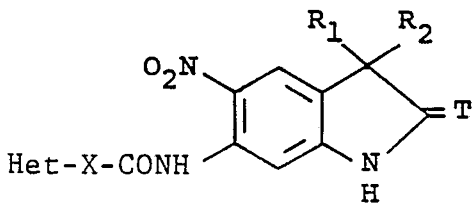

(VII)

in which $R_1$, $R_2$, T, X and Het have the above-given meanings, and subsequently cyclises; or

b) reacts a compound of the formula VIII

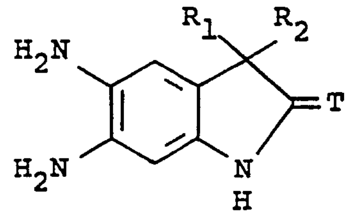

(VIII)

in which $R_1$, $R_2$ and T have the above-given meanings, with a compound of the formula IX

Het—X—CO—Y (IX)

in which Het and X have the above-given meanings and Y represents hydrogen or a reactive residue, and subsequently cyclises; or

c) cyclises a compound of the formula X

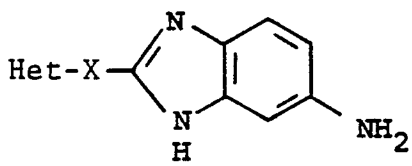

(X)

with appropriate ketones and sodium bisulphite or corresponding carboxylic acid hydrazides or carboxylic acid amides to the oxindole;

subsequently, if desired, converts compounds obtained of the formula I into other compounds of the formula I, as well as, if desired, converts the compounds with inorganic or organic acids into physiologically acceptable salts.

6. Process according to claim 5 for the preparation of compounds of the formula I, in which

$R_1$ signifies hydrogen, a methyl, ethyl, cyclopentyl or cyclohexyl group,

$R_2$ hydrogen, a methyl, ethyl, isopropyl, 3-pentyl, allyl, cyano, acetyl, propionyl, methoxycarbonyl, ethoxycarbonyl, aminocarbonyl, methylaminocarbonyl, dimethylaminocarbonyl and the hydrazinocarbonyl group or

$R_1$ and $R_2$, together with the C-atom to which they are attached, represent a spirocyclopropyl, spirocyclobutyl, spirocyclopentyl and spirocyclohexyl ring or $R_1$ and $R_2$ together represent an isopropylidene group,

X signifies a valency bond, a methylene or vinylene group,

T stands for an oxygen atom and

Het signifies a pyrrolyl, furanyl, thiophenyl, pyrazolyl, imidazolyl, thiazolyl, oxazolyl, triazolyl, tetrazolyl, thiadiazolyl, oxadiazolyl, pyrazinyl, N,N-dioxypyrazinyl, pyrimidinyl, N,N-dioxypyrimidinyl, pyridazinyl, oxazinyl, thiazinyl, triazinyl and the tetrazinyl radical, whereby the radicals Het can be substituted one or more times by hydroxyl, methyl, methoxy, methylmercapto, amino, nitro, halogeno or cyano.

7. Process according to claim 5 or 6 for the preparations of compounds of the formula I, in which

$R_1$ represents hydrogen, methyl, or ethyl,

$R_2$ methyl, ethyl or ethoxycarbonyl or

$R_1$ and $R_2$, together with the C-atom to which they are attached, a spirocyclopentane ring,

X signifies a valency bond or a $-CH_2$-group,

T represents oxygen and

Het is a furanyl, thienyl, pyrrolyl, thiazolyl, imidazolyl, triazolyl, oxazolyl, pyrazolyl, thiadiazolyl, pyrazinyl, pyridazinyl or pyrimidinyl ring, whereby the heterocycles can be substituted one or more times by hydroxyl or methyl.

8. Process according to claim 5, 6 or 7 for the preparation of the compounds of the formula I, in which

$R_1$ and $R_2$ signify the methyl or ethyl group,

X signifies a valency bond,

T represents oxygen and

Het a furanyl, thienyl, pyrrolyl, pyrazinyl, thiazolyl, pyridazinyl or pyrimidinyl ring.

9. Medicaments containing a compound according to claim 1, 2, 3 or 4, besides usual carrier and adjuvant materials.

10. Use of compounds according to claim 1, 2, 3, or 4 for the preparation of medicaments for the prophylaxis or treatment of heart and circulatory diseases.

11. Compounds of the formula VI

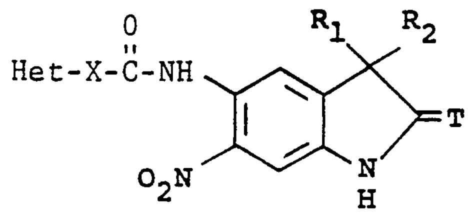

(VI)

in which $R_1$, $R_2$, X, T and Het have the meanings given in claim 1, their tautomers and their physiologically acceptable salts or inorganic and organic acids.

12. Compounds of the formula VII

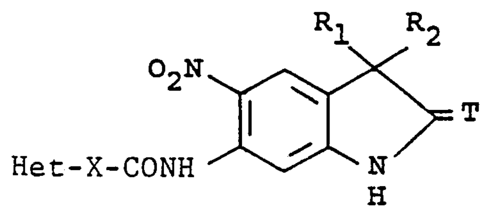

(VII)

in which $R_1$, $R_2$, X, T and Het have the meanings given in claim 1, their tautomers and their physiologically acceptable salts of inorganic and organic acids.